(19) Europäisches Patentamt / European Patent Office / Office européen des brevets

(11) EP 2 794 020 B1

(12) # EUROPÄISCHE PATENTSCHRIFT

(45) Veröffentlichungstag und Bekanntmachung des Hinweises auf die Patenterteilung:
**21.02.2018 Patentblatt 2018/08**

(21) Anmeldenummer: **12790875.4**

(22) Anmeldetag: **20.11.2012**

(51) Int Cl.:
*A61Q 5/06* (2006.01) *A61K 8/81* (2006.01)

(86) Internationale Anmeldenummer:
**PCT/EP2012/073098**

(87) Internationale Veröffentlichungsnummer:
**WO 2013/092075 (27.06.2013 Gazette 2013/26)**

(54) **MITTEL FÜR KERATINHALTIGE FASERN, ENTHALTEND MINDESTENS EIN SPEZIELLES AMPHIPHILES KATIONISCHES POLYMER UND MINDESTENS EIN POLYMER MIT STRUKTUREINHEITEN ABGELEITET VOM MALEINSÄUREESTER**

PRODUCT FOR KERATIN-CONTAINING FIBERS, CONTAINING AT LEAST ONE SPECIFIC AMPIPHILIC CATIONIC POLYMER AND AT LEAST A POLYMER WITH STRUCTURAL UNITS DERIVED FROM MALEIC ACID ESTER

PRODUITS DESTINÉS À DES FIBRES KÉRATINIQUES ET CONTENANT AU MOINS UN POLYMÈRE CATIONIQUE AMPHIPHILE SPÉCIAL ET AU MOINS UN POLYMÈRE POURVU DE MOTIFS STRUCTURAUX DÉRIVÉS DE L'ESTER D'ACIDE MALÉIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 DE 102011089564**

(43) Veröffentlichungstag der Anmeldung:
**29.10.2014 Patentblatt 2014/44**

(73) Patentinhaber: **Henkel AG & Co. KGaA**
**40589 Düsseldorf (DE)**

(72) Erfinder:
- **METTEN, Diane**
  **22393 Hamburg (DE)**
- **RICHTERS, Bernd**
  **21129 Hamburg (DE)**
- **SCHEFFLER, Rene**
  **25470 Ellerau (DE)**

(56) Entgegenhaltungen:
**WO-A1-99/22701 DE-A1-102008 038 105**

- **DATABASE GNPD [Online] MINTEL; Juni 2010 (2010-06), "Catalyst", XP002716840, Database accession no. 1357006**
- **DATABASE GNPD [Online] MINTEL; Mai 2006 (2006-05), "Styling Mousse", XP002716841, Database accession no. 532029**
- **DATABASE GNPD [Online] MINTEL; März 2011 (2011-03), "Re-Shaper Strong Hold Hairspray", XP002716842, Database accession no. 1511745**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Mittel zur Haarbehandlung, enthaltend eine Kombination mindestens eines speziellen amphiphilen, kationischen Polymers mit mindestens einem Polymer mit Struktureinheiten abgeleitet vom Maleinsäureester, die Verwendung dieser Mittel zur temporären Verformung und/oder zur Pflege keratinhaltiger Fasern und Aerosolhaarsprays/- schäume auf Basis dieser Mittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Stylingmittel zu entwickeln, die alle gewünschten Eigenschaften in Kombination aufweisen, bereitet nach wie vor Schwierigkeiten. Insbesondere gilt dies für die Kombination von starkem Halt einerseits und einfacher, gleichmäßiger Applikation auf die keratinhaltigen Fasern andererseits.

Mittel welche versuchen diese Zielstellung zu erfüllen sind zum Beispiel in den Patentanmeldungen DE 10 2008 038 105 A1 und WO 99/22701 A1 beschrieben.

Aufgabe der vorliegenden Erfindung war es daher, ein Mittel zur temporären Verformung und/oder zur Pflege keratinischer Fasern zur Verfügung zu stellen, das sich durch einen hohen Haltegrad bzw. durch eine hohe Pflegewirkung auszeichnet und insbesondere eine hervorragende Handhabbarkeit während der Applikation auf die keratinhaltigen Fasern besitzt.

Es wurde nunmehr überraschenderweise gefunden, dass dies durch eine Kombination spezieller Polymere erreicht werden kann. Ferner gelang es im Rahmen spezieller Ausführungsformen der Erfindung zusätzlich zu diesen hervorragenden Eigenschaften Zusammensetzungen ohne Trübung bereitzustellen. Die Trübungsfreiheit ist insbesondere im Rahmen der Bereitstellung von Aerosolzusammensetzungen von Belang, da feste Schwebeteilchen zu einer Verstopfung der Austrittsdüse der Aerosolverpackung führen können. Generell besteht bei trüben und dünnflüssigen Zusammensetzungen zusätzlich die Gefahr der Sedimentation, die sich nachteilig auf die Lagerstabilität der Zusammensetzung auswirkt.

Ein erster Gegenstand der vorliegenden Erfindung ist daher ein kosmetisches Mittel zur kosmetischen Behandlung keratinhaltiger Fasern, insbesondere menschlichem Haar, enthaltend in einem kosmetisch akzeptablen Träger

(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV),

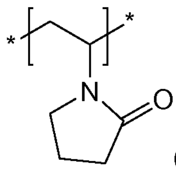 (I) 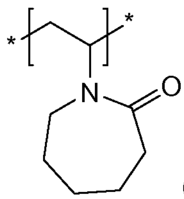 (II) 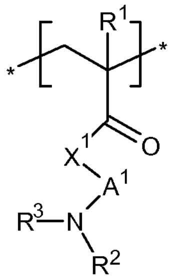 (III) 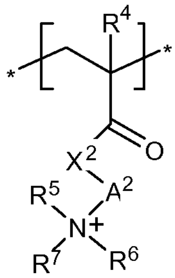 (IV)

worin

$R^1$ und $R^4$ stehen unabhängig voneinander für ein Wasserstoffatom oder eine Methylgruppe,
$X^1$ und $X^2$ stehen unabhängig voneinander für ein Sauerstoffatom oder eine Gruppe NH,
$A^1$ und $A^2$ stehen unabhängig voneinander für eine Gruppe Ethan-1,2-diyl, Propan-1,3-diyl oder Butan-1,4-diyl,
$R^2$, $R^3$, $R^5$ und $R^6$ stehen unabhängig voneinander für eine ($C_1$ bis $C_4$)-Alkylgruppe,
$R^7$ steht für eine ($C_8$ bis $C_{30}$)-Alkylgruppe
und

(b) mindestens ein Copolymer umfassend mindestens eine Struktureinheit der Formel (A1) und mindestens eine Struktureinheit der Formel (A2)

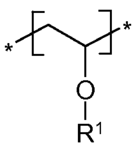 (A1) 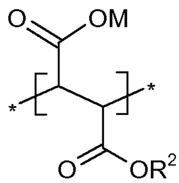 (A2)

worin

$R^1$ steht für eine ($C_1$ bis $C_{18}$)-Alkylgruppe,
$R^2$ steht für eine ($C_1$ bis $C_6$)-Alkylgruppe,
M steht für ein Wasserstoffatom oder ein Äquivalent eines ein- oder mehrwertigen Kations

(c) mindestens ein Treibmittel.

Gemäß obiger Formeln und allen folgenden Formeln steht eine chemische Bindung, die mit dem Symbol * gekennzeichnet ist, für eine freie Valenz des entsprechenden Strukturfragments.

Zur Kompensation der positiven Polymerladung in dem erfindungsgemäß Mittel dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Beispiele für erfindungsgemäße ($C_1$ bis $C_4$)-Alkylgruppen sind Methyl, Ethyl, Propyl, Isopropyl, Butyl, Sec-Butyl, Isobutyl, tert-Butyl.

Beispiele für erfindungsgemäße ($C_8$ bis $C_{30}$)-Alkylgruppen sind Octyl (Capryl), Decyl (Caprinyl), Dodecyl (Lauryl), Tetradecyl (Myristyl), Hexadecyl (Cetyl), Octadecyl (Stearyl), Eicosyl (Arachyl), Docosyl (Behenyl).

Beispiele für ($C_4$ bis $C_{12}$)-Alkylaminocarbonylgruppen sind Butylaminocarbonyl, Sec-Butylaminocarbonyl, Isobutylaminocarbonyl, tert-Butylaminocarbonyl, (2,4,4-Trimethylpent-2-yl)aminocarbonyl, Neopentylaminocarbonyl, 2-Ethylhexylaminocarbonyl, Neodecylaminocarbonyl. Beispiele für ($C_4$ bis $C_{12}$)-Alkylaminoethylaminocarbonylgruppen sind Butylaminoethylaminocarbonyl, Sec-Butylaminoethylaminocarbonyl, Isobutylaminoethylaminocarbonyl, tert-Butylaminoethylaminocarbonyl, (2,4,4-Trimethylpent-2-yl)aminoethylaminocarbonyl, Neopentylaminoethylaminocarbonyl, 2-Ethylhexylaminoethylaminocarbonyl, Neodecylaminoethylaminocarbonyl.

Beispiele für ($C_4$ bis $C_{12}$)-Alkylaminopropylaminocarbonylgruppen sind Butylaminopropylaminocarbonyl, Sec-Butylaminopropylaminocarbonyl, Isobutylaminopropylaminocarbonyl, tert-Butylaminopropylaminocarbonyl, (2,4,4-Trimethylpent-2-yl)aminopropylaminocarbonyl, Neopentylaminopropylaminocarbonyl, 2-Ethylhexylaminopropylaminocarbonyl, Neodecylaminopropylaminocarbonyl.

Beispiele für ($C_4$ bis $C_{12}$)-Alkyloxycarbonylgruppen sind Butyloxycarbonyl, Sec-Butyloxycarbonyl, Isobutyloxycarbonyl, tert-Butyloxycarbonyl, (2,4,4-Trimethylpent-2-yl)oxycarbonyl, Neopentyloxycarbonyl, 2-Ethylhexyloxycarbonyl, Neodecyloxycarbonyl.

Beispiele für ($C_4$ bis $C_{12}$)-Alkylaminoethyloxycarbonylgruppen sind Butylaminoethyloxycarbonyl, Sec-Butylaminoethyloxycarbonyl, Isobutylaminoethyloxycarbonyl, tert-Butylaminoethyloxycarbonyl, (2,4,4-Trimethylpent-2-yl)aminoethyloxycarbonyl, Neopentylaminoethyloxycarbonyl, 2-Ethylhexylaminoethyloxycarbonyl, Neodecylaminoethyloxycarbonyl.

Beispiele für ($C_4$ bis $C_{12}$)-Alkylaminopropyloxycarbonylgruppen sind Butylamino-propyloxycarbonyl, Sec-Butylaminopropyloxycarbonyl, Isobutylaminopropyloxycarbonyl, tert-Butylaminopropyloxycarbonyl, (2,4,4-Trimethylpent-2-yl)aminopropyloxycarbonyl, Neopentylaminopropyloxycarbonyl, 2-Ethylhexylaminopropyloxycarbonyl, Neodecylaminopropyloxycarbonyl. Beispiele für erfindungsgemäße ($C_4$ bis $C_{12}$)-Alkylgruppen sind Butyl, Sec-Butyl, Isobutyl, tert-Butyl, 2,4,4-Trimethylpent-2-yl, Neopentyl, 2-Ethylhexyl, Neodecyl.

Beispiele für ($C_2$ bis $C_{12}$)-Acyloxygruppen sind Acetoxy, Propionyloxy und Neodecanoyloxy.

Im Sinne der Erfindung bevorzugte Mittel enthalten die amphiphilen, kationischen Polymere (a) in einer Menge von 0,05 Gew.-% bis 8,0 Gew.-%, besonders bevorzugt von 0,1 Gew.-% bis 5,0 Gew.-%, ganz besonders bevorzugt von 0,2 bis 2,5 Gew.%, jeweils bezogen auf das Gesamtgewicht des Mittels.

Bevorzugte Mittel enthalten die Copolymere (b) in einer Menge von 0,05 Gew.-% bis 8,0 Gew.-%, besonders bevorzugt von 0,1 Gew.-% bis 5,0 Gew.-%, ganz besonders bevorzugt von 0,2 bis 2,5 Gew.%, jeweils bezogen auf das Gesamtgewicht des Mittels.

Ein bevorzugt geeignetes kosmetisches Mittel dieser Erfindung enthält die amphiphilen, kationischen Polymere der Komponente (a) und die Copolymere der Komponente (b) in einem Gewichtsverhältnisbereich von (a) zu (b) von 5 zu 1 bis 1 zu 5, insbesondere von 2 zu 1 bis 1 zu 2.

Die erfindungsgemäßen amphiphilen, kationischen Polymere weisen bevorzugt ein Molekulargewicht von 10000 g/mol bis 50000000 g/mol, insbesondere von 50000 g/mol bis 5000000 g/mol, besonders bevorzugt von 75000 g/mol bis 1000000 g/mol, auf.

Besonders vorteilhaft erweisen sich die Eigenschaften des erfindungsgemäßen Mittels, wenn es als Aerosolspray, Aerosolschaum, Pumpspray oder Pumpschaum konfektioniert wird. Diese bevorzugte Form der Konfektionierung wird später im Detail beschrieben.

Nachfolgende amphiphile, kationische Polymere (a) finden erfindungsgemäß bevorzugt in den erfindungsgemäßen Mitteln Einsatz, wenn die amphiphilen, kationischen Polymere (a) bezüglich oben genannter Formeln (I) bis (IV) eines oder mehrere der folgenden Merkmale erfüllen:

- $R^1$ und $R^4$ bedeuten jeweils eine Methylgruppe,
- $X^1$ steht für eine Gruppe NH,
- $X^2$ steht für eine Gruppe NH,
- $A^1$ und $A^2$ stehen unabhängig voneinander für Ethan-1,2-diyl oder Propan-1,3-diyl,
- $R^2$, $R^3$, $R^5$ und $R^6$ stehen unabhängig voneinander für Methyl oder Ethyl, (besonders bevorzugt für Methyl),
- $R^7$ steht für eine ($C_{10}$ bis $C_{24}$)-Alkylgruppe, insbesondere für Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

Es ist erfindungsgemäß bevorzugt, die Struktureinheit der Formel (III) aus aus mindestens einer Struktureinheit der Formel (III-1) bis (III-8) auszuwählen

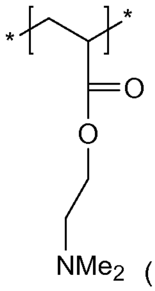 (III-1) 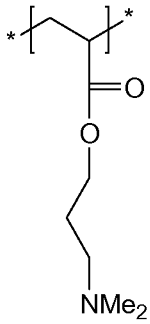 (III-2) 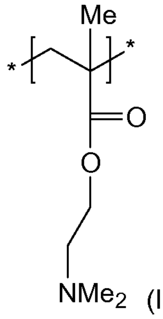 (III-3) 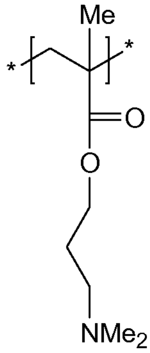 (III-4)

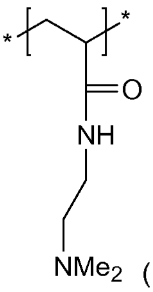 (III-5) 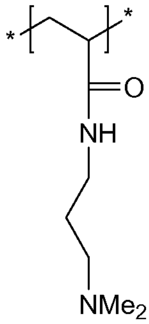 (III-6) 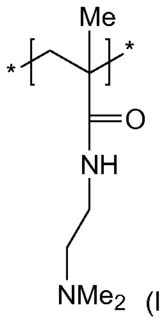 (III-7) 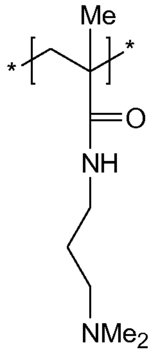 (III-8).

Außerdem erweist es sich als besonders bevorzugt, als Struktureinheit der Formel (III) die Struktureinheit gemäß Formel (III-7) und/oder der Formel (III-8) zu wählen. Die Struktureinheit der Formel (III-8) ist erfindungsgemäß eine ganz besonders bevorzugte Struktureinheit.

Ferner stellte es sich mit Blick auf die Lösung der Aufgabe als bevorzugt heraus, wenn die Struktureinheit der Formel (IV) ausgewählt wird, aus mindestens einer Struktureinheit der Formeln (IV-1) bis (IV-8)

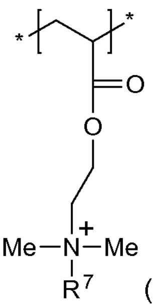 (IV-1) 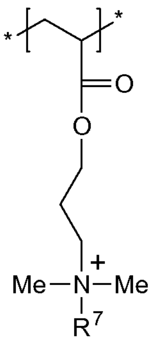 (IV-2) 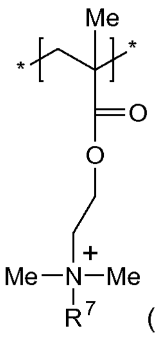 (IV-3) 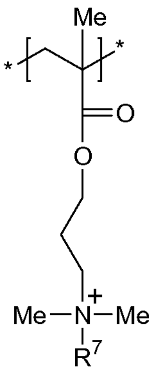 (VI-4)

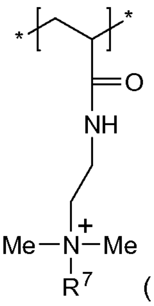 (IV-5) 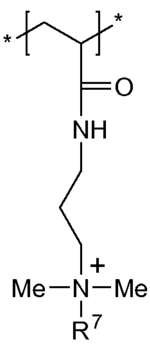 (IV-6) 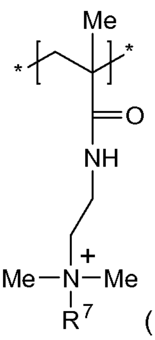 (IV-7) 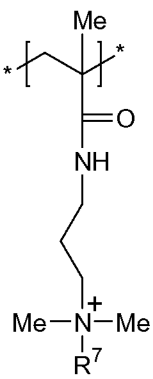 (VI-8),

worin $R^7$ jeweils für eine ($C_8$ bis $C_{30}$)-Alkylgruppe steht.

Als wiederum besonders bevorzugte Struktureinheit der Formel (IV) gelten die Struktureinheiten der Formel (IV-7) und/oder der Formel (IV-8), worin jeweils $R^7$ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl). Die Struktureinheit der Formel (IV-8) stellt erfindungsgemäß eine ganz besonders bevorzugte Struktureinheit der Formel (IV) dar.

Ein ganz besonders bevorzugt in dem erfindungsgemäßen Mittel enthaltenes amphiphiles, kationisches Polymer umfasst mindestens eine Struktureinheit der Formel (II), mindestens eine Struktureinheit der Formel (III-8) und mindestens eine Struktureinheit der Formel (IV-8),

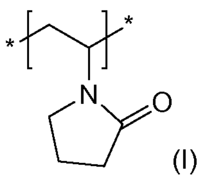
(I)
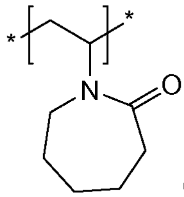
(II)
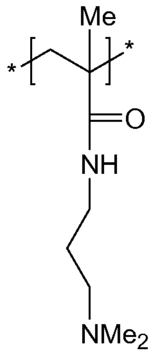
(III-8)
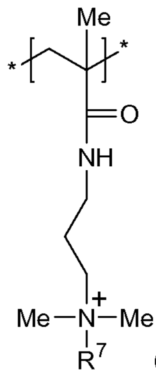
(IV-8)

worin $R^7$ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

Ein ganz besonders bevorzugtes erfindungsgemäßes amphiphiles, kationisches Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle® 300 (30 Gew.-% Aktivsubstanz in Ethanol-Wasser-Gemisch, Molekulargewicht 350000) von der Firma ISP vertrieben wird.

Das erfindungsgemäße Mittel enthält neben dem besagten amphiphilen, kationischen Polymer der Komponente (a) zwingend als Komponente (b) mindestens ein zuvor definiertes Polymer mit Struktureinheiten abgeleitet vom Maleinsäureester (*vide supra*). Dieses besagte Polymer ist selbstredend von den Verbindungen der Komponente (a) verschieden.

Es ist erfindungsgemäß bevorzugt, wenn $R^1$ gemäß Formel (A1) für eine Methylgruppe steht.

Es ist erfindungsgemäß bevorzugt, wenn $R^2$ gemäß Formel (A2) für eine ($C_2$ bis $C_4$)-Alkylgruppe steht, insbesondere für Ethyl, n-Propyl, Isopropyl, n-Butyl oder tert-Butyl.

Wenn die Struktureinheiten der Formel (A2) mit einer Carbonsäurefunktion vorliegen, bedeutet der Rest M ein Wasserstoffatom. Wenn die Struktureinheiten der Formel (A2) als Salz der Carbonsäure vorliegen, stehen M für ein Äquivalent eines ein- oder mehrwertigen Kations. Das ein oder mehrwertige Kation $M^{z+}$ mit jeweils einer Ladungszahl z von eins oder höher dient lediglich aus Gründen der Elektroneutralität zur Kompensation der einfach negativen Ladung des bei Salzbildung vorliegenden Carboxylatfragments $-COO^{(-)}$ der Formel (A2) . Das dafür zu verwendende Äquivalent des entsprechenden Kations beträgt 1/z. Das Fragment -COOM der Formel (A2) steht im Fall der Salzbildung für die Gruppe:

$$-COO^{(-)}\ 1/z\ (M^{z+})$$

Als ein- oder mehrwertige Kationen $M^{z+}$ kommen prinzipiell alle physiologisch verträglichen Kationen in Frage. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine (insbesondere ($C_2$ bis $C_6$)-Alkanolaminen (*vide infra*: Neutralisierung der besagten Polymere mit ($C_2$ bis $C_6$)-Alkanolaminen) mit einer Säure, wie z.B. mit Struktureinheiten der Formel (A2) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht in der Formel (A2) bevorzugt für ein Wasserstoffatom, ein Ammoniumion, ein Alkalimetallion oder ein Hydroxy-($C_2$ bis $C_6$)-alkylammoniumion, besonders bevorzugt für ein Wasserstoffatom, ein Ammoniumion, ein Hydroxy-($C_2$ bis $C_6$)-alkylammoniumion, ein Natriumion oder ein Kaliumion.

Es ist erfindungsgemäß bevorzugt, wenn die erfindungsgemäßen Mittel als Komponete (b) mindestens ein Polymer enthalten, ausgewählt aus mindestens einem Polymer der Gruppe mit Polymeren der INCI-Nomenklatur Butyl Ester of PVM/MA Copolymer, Isopropyl Ester of PVM/MA Copolymer, Ethyl Ester of PVM/MA Copolymer.

Entsprechende Polymere der Komponete (b) des erfindungsgemäßen Mittels werde beispielsweise unter dem Handelsnamen Gantrez® ES 425 (Copolymer von Methylvinylether und dem Butyl-Halbester der Maleinsäure; 50 Gew.-% Aktivsubstanz in Ethanol; INCI-Bezeichnung: Butyl Ester of PVM/MA-Copolymer (Ashland)), Gantrez® ES 435 (Copolymer von Methylvinylether und dem Butyl-Halbester der Maleinsäure; 50 Gew.-% Aktivsubstanz in Isopropanol; INCI-Bezeichnung: Butyl Ester of PVM/MA-Copolymer (Ashland)), Gantrez® ES 3351 (Copolymer von Methylvinylether und dem Isopropyl-Halbester der Maleinsäure; 50 Gew.-% Aktivsubstanz in Isopropanol; INCI-Bezeichnung: Butyl Ester of PVM/MA-Copolymer (Ashland)), Gantrez® ES 225 (Copolymer von Methylvinylether und dem Ethyl-Halbester der Maleinsäure; 50 Gew.-% Aktivsubstanz in Ethanol) INCI-Bezeichnung: Ethyl Ester of PVM/MA-Copolymer (Ashland)).

Folgende Ausführungsformen der erfindungsgemäßen kosmetischen Mittel eignen sich zur Lösung der Aufgabe ganz besonders gut:

(A) Mittel zur kosmetischen Behandlung keratinhaltiger Fasern, insbesondere menschlichem Haar, enthaltend in einem kosmetisch akzeptablen Träger

(a) mindestens ein amphiphiles, kationisches Polymer umfassend mindestens eine Struktureinheit der Formel (I), mindestens eine Struktureinheit der Formel (II), mindestens eine Struktureinheit der Formel (III-8) und mindestens eine Struktureinheit der Formel (IV-8),

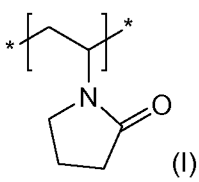 (I) 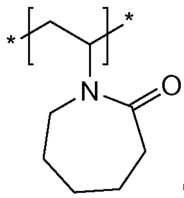 (II) 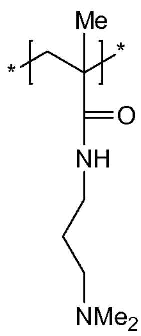 (III-8) 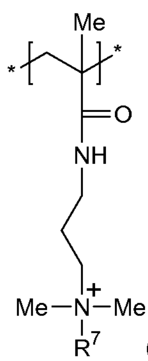 (IV-8)

worin $R^7$ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).
und
(b) mindestens ein Copolymer umfassend mindestens eine Struktureinheit der Formel (A1) und mindestens eine Struktureinheit der Formel (A2)

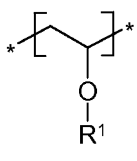 (A1) 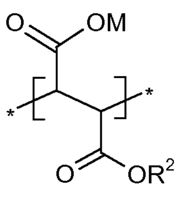 (A2)

worin

- $R^1$ steht für eine Methylgruppe,
- $R^2$ steht für eine Ethylgruppe, eine n-Propylgruppe, eine Isopropylgruppe, eine n-Butylgruppe oder eine tert-Butylgruppe,
- M steht für ein Wasserstoffatom oder ein Äquivalent eines ein- oder mehrwertigen Kations.

(B) Mittel zur kosmetischen Behandlung keratinhaltiger Fasern, insbesondere menschlichem Haar, enthaltend in einem kosmetisch akzeptablen Träger

(a) mindestens ein amphiphiles, kationisches Polymer mit der INCI-Nomenklatur Polyquaternium-69
und
(b) mindestens ein Copolymer ausgewählt aus mindestens einem Polymer der Gruppe mit Polymeren der INCI-Nomenklatur Butyl Ester of PVM/MA Copolymer, Isopropyl Ester of PVM/MA Copolymer, Ethyl Ester of PVM/MA Copolymer.

(C) Mittel zur kosmetischen Behandlung keratinhaltiger Fasern, insbesondere menschlichem Haar, enthaltend in einem kosmetisch akzeptablen Träger

(a) 0,05 Gew.-% bis 8,0 Gew.-%, bevorzugt von 0,1 Gew.-% bis 5,0 Gew.-%, besonders bevorzugt von 0,2 bis 2,5 Gew.%, mindestens eines amphiphilen, kationischen Polymers umfassend mindestens eine Struktureinheit der Formel (I), mindestens eine Struktureinheit der Formel (II), mindestens eine Struktureinheit der Formel (III-8) und mindestens eine Struktureinheit der Formel (IV-8),

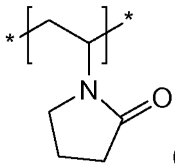 (I) 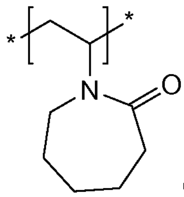 (II) 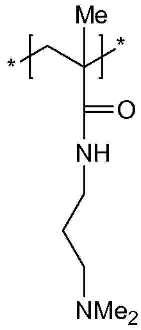 (III-8) 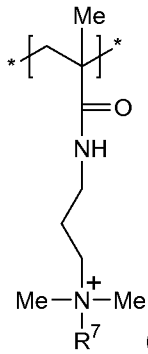 (IV-8)

worin $R^7$ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).
und
(b) 0,05 Gew.-% bis 8,0 Gew.-%, bevorzugt von 0,1 Gew.-% bis 5,0 Gew.-%, besonders bevorzugt von 0,2 bis 2,5 Gew.%, mindestens eines Copolymers umfassend mindestens eine Struktureinheit der Formel (A1) und mindestens eine Struktureinheit der Formel (A2)

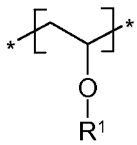 (A1) 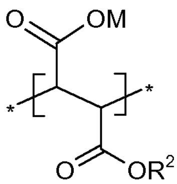 (A2)

worin

- $R^1$ steht für eine Methylgruppe,
- $R^2$ steht für eine Ethylgruppe, eine n-Propylgruppe, eine Isopropylgruppe, eine n-Butylgruppe oder eine tert-Butylgruppe,
- M steht für ein Wasserstoffatom oder ein Äquivalent eines ein- oder mehrwertigen Kations.

(D) Mittel zur kosmetischen Behandlung keratinhaltiger Fasern, insbesondere menschlichem Haar, enthaltend in einem kosmetisch akzeptablen Träger

(a) 0,05 Gew.-% bis 8,0 Gew.-%, bevorzugt von 0,1 Gew.-% bis 5,0 Gew.-%, besonders bevorzugt von 0,2 bis 2,5 Gew.%, mindestens eines amphiphilen, kationischen Polymers mit der INCI-Nomenklatur Polyquaternium-69
und
(b) 0,05 Gew.-% bis 8,0 Gew.-%, bevorzugt von 0,1 Gew.-% bis 5,0 Gew.-%, besonders bevorzugt von 0,2 bis 2,5 Gew.%, mindestens eines Copolymers ausgewählt aus mindestens einem Polymer der Gruppe mit Polymeren der INCI-Nomenklatur Butyl Ester of PVM/MA Copolymer, Isopropyl Ester of PVM/MA Copolymer, Ethyl Ester of PVM/MA Copolymer.

Dabei ist es besonders bevorzugt, wenn für alle Ausführungsformen dieser ersten Ausführungsform, wenn die Copolymere der Komponente (b) ganz oder teilweise neutralisiert in der Salzform vorliegen. Zur Neutralisation wird bevorzugt mindestens ein ($C_2$ bis $C_6$)-Alkanolamin verwendet. Aus diesem Grunde enthalten bevorzugte erfindungsgemäße Mittel (insbesondere die bevorzugten Ausführungsformen (A) bis (D)) zusätzlich mindestens ein ($C_2$ bis $C_6$)-Alkanolamin. Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Die erfindungsgemäßen Mittel weisen bevorzugt bei 20°C einen pH-Wert von pH 4 bis pH 9, besonders bevorzugt von pH 6 bis pH 7 auf.

Zur Intensivierung des erfindungsgemäßen Effektes enthalten die erfindungsgemäßen Mittel vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.

Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR^2)_wOR^3 \qquad \text{(E4-I)}$$

  in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$$R^4O\text{-}[G]_p \qquad \text{(E4-II)}$$

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside $C_{12}$-$C_{30}$-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin® CS 15 (COGNIS), Cremophor A25 (BASF SE) bzw. Eumulgin® CS 50 (COGNIS) vermarktet werden.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel $R-O-(CH_2-CH_2O)_x-CH_2-COOH$, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R-O(CH_2-CH_2O)_x-OSO_3H$, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- sulfatierte Fettsäurealkylenglykolester der Formel (E1-II)

  $$R^7CO(AlkO)_nSO_3M \qquad \text{(E1-II)}$$

  in der $R^7CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906 beschrieben sind,
- Amidethercarbonsäuren,
- Kondensationsprodukte aus $C_8$ - $C_{30}$ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer $C_8$ - $C_{24}$- Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das $C_{12}$ - $C_{18}$ -Acylsarcosin.

Die erfindungsgemäßen Mittel enthalten die Inhalts- bzw. Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Es ist erfindungsgemäß bevorzugt mindestens einen ($C_1$ bis $C_4$)-Monoalkylalkohol in den erfindungsgemäßen Mitteln insbesondere in einer Menge von 1 bis 50 Gew.-% insbesondere von 5 bis 30 Gew.-% einzusetzen. Dies ist wiederum insbesondere für die Konfektionierung als Pumpschaum oder Aerosolschaum bevorzugt.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Siliciumorganischer Verbindungen. Zunächst werden hierunter die Dimethiconole verstanden.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company,

Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole (S3) bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning® 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 $\mu$m, bevorzugt 0,01 bis 100 $\mu$m, besonders bevorzugt 0,01 bis 20 $\mu$m und ganz besonders bevorzugt 0,01 bis 10 $\mu$m. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäßen Mittel zusätzlich mindestens ein aminofunktionelles Silikon enthalten. Darunter sind Silikone zu verstehen, welche mindestens eine, gegebenenfalls substituierte, Aminogruppe aufweisen. Diese Silikone werden nach der INCI-Deklaration als Amodimethicone bezeichnet und sind beispielsweise in Form einer Emulsion als Handelsprodukt Dow Corning® 939 oder als Handelsprodukt Dow Corning® 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Sericin (Pentapharm) und Kerasol® (Croda) vertrieben.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a. Vitamin $B_1$ (Thiamin), Vitamin $B_2$ (Riboflavin), Vitamin $B_3$ (Nicotinsäure und/oder Nicotinsäureamid (Niacinamid)), Vitamin $B_5$ (Pantothensäure, Panthenol und Pantolacton), Vitamin $B_6$ (Pyridoxin sowie Pyridoxamin und Pyridoxal), Vitamin C (Ascorbinsäure), Vitamin E (Tocopherole, insbesondere α-Tocopherol), Vitamin F (Linolsäure und/oder Linolensäure), Vitamin H.

Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weiterhin kann es bevorzugt sein, in den erfindungsgemäßen Mitteln Mischungen aus mehreren, insbesondere aus zwei, verschiedenen Pflanzenextrakten einzusetzen.

Auch Mono- bzw. Oligosaccharide können als Pflegestoff in den erfindungsgemäßen Mitteln eingesetzt werden.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist. Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I),

$$CH_2O(CH_2CH_2O)_mR^1$$
$$|$$
$$CHO(CH_2CH_2O)_nR^2 \qquad (D4\text{-}I)$$
$$|$$
$$CH_2O(CH_2CH_2O)_qR^3$$

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbare UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, $\alpha$-(2-Oxoborn-3-yliden)-toluol-4-sulfonsäure und deren Salze, ethoxylierte 4-Aminobenzoesäure-ethylester (PEG-25 PABA; Uvinul®P 25), 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz (Benzophenone-4; Uvinul®MS 40; Uvasorb®S 5), 3-(4'-Methylbenzyliden)-D,L-Campher, 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon, 1,1'-Diphenylacrylonitrilsäure-2-ethylhexyl-ester, o-Aminobenzoesäurementhylester, 2,2',4,4'-Tetrahydroxybenzophenon, 2,2'-Dihydroxy-4,4'-dimethoxybenzophenon, 2,2'-Dihydroxy-4,4'-dimethoxybenzophenon-5-natriumsulfonat und 2-Cyano-3,3-diphenylacrylsäure-2'-ethylhexylester. Bevorzugt sind 2-Hydroxy-4-methoxybenzophenon-5-sulfonsäure und deren Natriumsalz und/oder ethoxylierte 4-Aminobenzoesäure-ethylester.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-($\beta$-hydroxyethyl)-amino-2-nitrobenzol, 3-Nitro-4-($\beta$-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugt werden kationische direktziehende Farbstoffe eingesetzt. Besonders bevorzugt sind dabei

(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Farbstoffe, die auch unter den Bezeichnungen Basic Yellow 87, Basic Orange 31 und Basic Red 51 bekannt

sind, sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Vorzugsweise werden die erfindungsgemäßen Mittel als Pumpspray, Aerosolspray, Pumpschaum oder Aerosolschaum.

Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird.

Insbesondere bevorzugt sind die erfindungsgemäßen Mittel als Aerosolhaarschaum oder Aerosolhaarspray konfektioniert. Das erfindungsgemäße Mittel (insbesondere die bevorzugten Ausführungsformen (A) bis (F) (*vide supra*)) enthält daher bevorzugt zusätzlich mindestens ein Treibmittel.

Erfindungsgemäß geeignete Treibmittel sind beispielsweise ausgewählt aus $N_2O$, Dimethylether, $CO_2$, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus $N_2O$, Dimethylether, $CO_2$, Luft, Alkanen mit 3 oder 4 Kohlenstoffatomen (wie Propan, n-Butan, iso-Butan) sowie Mischungen daraus.

Eine bevorzugte Ausführungsform der erfindungsgemäßen Mittel sind Aerosolhaarschäume oder Aerosolhaarsprays, enthaltend das zuvor beschriebene erfindungsgemäße Mittel und mindestens ein Treibmittel.

Bevorzugte erfindungsgemäße Mittel und Treibmittel des Aerosolhaarschaums bzw. Aerosolhaarsprays, sowie die jeweiligen Mengen an Treibmittel entsprechen dem bereits oben Ausgeführten.

Ein zweiter Gegenstand der Erfindung ist die Verwendung der erfindungsgemäßen Mittel zur temporären Verformung von Haaren und/oder zur Haarpflege.

Die erfindungsgemäßen Mittel und Produkte, die diese Mittel enthalten, insbesondere Aerosolhaarschäume bzw. Aerosolhaarsprays, zeichnen sich insbesondere dadurch aus, dass sie behandeltem Haar einen sehr starken, dauerhaften Frisurenhalt verleihen, obgleich das Haar bleibt flexibel bleibt. Wird das Mittel als Haarschaum konfektioniert, bildet sich ein stabiler, feinporiger und cremiger Schaum, der sich gleichmäßig und ohne zu tropfen auf dem Haar verteilen lässt.

Ein dritter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand zu einem Schaum verschäumt wird und der resultierende Schaum auf die keratinhaltigen Fasern appliziert wird.

Dabei ist es erfindungsgemäß bevorzugt, dass die keratinhaltigen Fasern in Form gebracht werden und diese Form durch das Mittel des ersten Erfindungsgegenstandes fixiert wird.

Als erfindungsgemäß bevorzugt gelten die zuvor genannten Abgabevorrichtungen (*vide supra*).

Ein vierter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand als Spray auf die keratinhaltigen Fasern appliziert wird.

Dabei ist es erfindungsgemäß bevorzugt, dass die keratinhaltigen Fasern in Form gebracht werden und diese Form durch das Mittel des ersten Erfindungsgegenstandes fixiert wird.

Als erfindungsgemäß bevorzugt gelten die zuvor genannten Abgabevorrichtungen (*vide supra*).

Beispiele

Die folgenden Mengenangaben verstehen sich - soweit nichts anderes vermerkt ist - in Gewichtsprozent.

Folgende Rezepturen wurden durch Vermischen der angegebenen Rohstoffe bereitgestellt:

| Rohstoffe | E1 | V1 | V2 |
|---|---|---|---|
| 2-Amino-2-methylpropan-1-ol | 0,067 | - | 0,134 |
| Aquastyle® 300 [1] | 2,50 | 5,00 | - |
| Gantrez ES 425 [2] | 1,50 | - | 3,00 |
| Wasser | <--------------------- ad 100 ---------------------> | | |

[1] INCI-Bezeichnung: Polyquaternium-69 (30 Gew.-% Aktivsubstanz in Ethanol-Wasser-Gemisch, Molekulargewicht 350000) (Ashland),
[2] Copolymer von Methylvinylether und dem Butyl-Halbester der Maleinsäure (50 Gew.-% Aktivsubstanz in Ethanol) INCI-Bezeichnung: Butyl Ester of PVM/MA-Copolymer (Ashland)

Es wurden standardisierte Haarsträhnen der Fa. Kerling (Art. Nr. 827560) des Haartyps "European Natural, Farbe 6/0) von einer Länge ($L_{max}$) von 220 mm und einem Gewicht von 0.6 g eingesetzt. Zur Vorbereitung wurden die Strähnen mit einer 12.5 Gew.-%igen Natriumlaurethsulphat-Lösung gewaschen. Die Haarsträhnen wurden über Nacht in einem Trockenofen bei 318 K getrocknet.

0.18g der Zusammensetzungen wurden auf jeweils eine Haarsträhne appliziert und einmassiert. Die Strähne wurde sodann auf einen Wickler gewickelt (Fripac-medis, Ø 7mm, Art. Nr. D-1203) und über Nacht bei Raumtemperatur getrocknet.

Die Wickler wurden vorsichtig entfernt und die Strähne aufgehängt. Die Längen der Locken wurden jeweils gemessen ($L_0$) und die Strähnen in eine Klimakammer gegeben. Dort wurden sie bei 294 K und einer relativen Luftfeuchtigkeit von 85% über einen Zeitraum von 24 h gelagert und danach die Längen der Locken erneut vermessen ($L_t$).

Pro Zusammensetzung wurden 5 Teststrähnen entsprechend behandelt und vermessen.

Die High-Humidity-Curlretention (HHCR) wurde nach folgender Formel berechnet und für jede Zusammensetzung das arithmetische Mittel aus den HHCR-Werten der 5 Teststrähnen gebildet:

$$HHCR = \frac{L_{\max} - L_t}{L_{\max} - L_0}$$

| | |
|---|---|
| HHCR E1: | 97 % |
| HHCR V1: | 82 % |
| HHCR V2: | 29 % |

Die Polymerkombination des erfindungsgemäßen Mittels E1 (insgesamt 1,5 Gew.-% Aktivsubstanz an Polymer) weist eine signifikant höhere HHCR auf, als die gleiche Polymermenge (1,5 Gew.-% Aktivsubstanz) des jeweils einzelnen Polymers der Vergleichszusammensetzungen V1 und V2.

## Patentansprüche

1. Kosmetisches Mittel zur kosmetischen Behandlung keratinhaltiger Fasern, insbesondere menschlichem Haar, enthaltend in einem kosmetisch akzeptablen Träger

(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV),

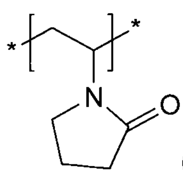
(I)
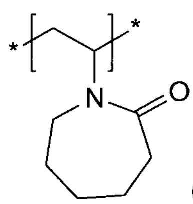
(II)
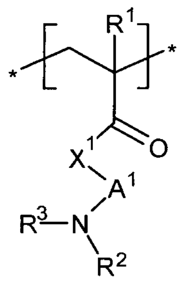
(III)
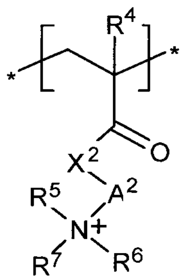
(IV)

worin

$R^1$ und $R^4$ stehen unabhängig voneinander für ein Wasserstoffatom oder eine Methylgruppe,
$X^1$ und $X^2$ stehen unabhängig voneinander für ein Sauerstoffatom oder eine Gruppe NH,
$A^1$ und $A^2$ stehen unabhängig voneinander für eine Gruppe Ethan-1,2-diyl, Propan-1,3-diyl oder Butan-1,4-diyl,
$R^2$, $R^3$, $R^5$ und $R^6$ stehen unabhängig voneinander für eine ($C_1$ bis $C_4$)-Alkylgruppe, $R^7$ steht für eine ($C_8$ bis $C_{30}$)-Alkylgruppe
und

(b) mindestens ein Copolymer umfassend mindestens eine Struktureinheit der Formel (A1) und mindestens eine Struktureinheit der Formel (A2)

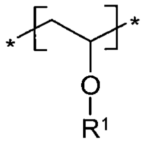
(A1)
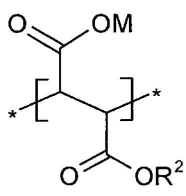
(A2)

worin

$R^1$ steht für eine ($C_1$ bis $C_{18}$)-Alkylgruppe,
$R^2$ steht für eine ($C_1$ bis $C_6$)-Alkylgruppe,
M steht für ein Wasserstoffatom oder ein Äquivalent eines ein- oder mehrwertigen Kations

(c) mindestens ein Treibmittel.

2. Mittel nach Anspruch 1, **dadurch gekennzeichnet, dass** gemäß Formel (III) beziehungsweise Formel (IV) $R^1$ und $R^4$ jeweils eine Methylgruppe bedeuten.

3. Mittel nach einem der Ansprüche 1 oder 2, **dadurch gekennzeichnet, dass** gemäß Formel (III) beziehungsweise Formel (IV) $A^1$ und $A^2$ stehen unabhängig voneinander für Ethan-1,2-diyl oder Propan-1,3-diyl.

4. Mittel nach einem der Ansprüche 1 bis 3, **dadurch gekennzeichnet, dass** gemäß Formel (III) beziehungsweise Formel (IV) $R^2$, $R^3$, $R^5$ und $R^6$ stehen unabhängig voneinander für Methyl oder Ethyl, (besonders bevorzugt für Methyl).

5. Mittel nach einem der Ansprüche 1 bis 4, **dadurch gekennzeichnet, dass** gemäß Formel (IV) $R^7$ steht für eine ($C_{10}$ bis $C_{24}$)-Alkylgruppe, insbesondere für Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

6. Mittel nach einem der Ansprüche 1 bis 5, **dadurch gekennzeichnet, dass** das darin enthaltene amphiphile, kationische Polymer mindestens eine Struktureinheit der Formel (II), mindestens eine Struktureinheit der Formel (III-8) und mindestens eine Struktureinheit der Formel (IV-8) umfasst

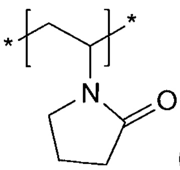 (I) 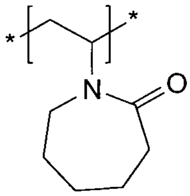 (II) 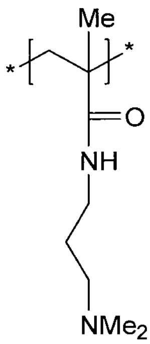 (III-8) 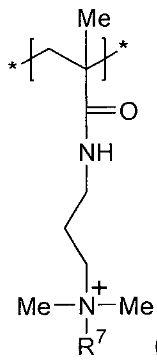 (IV-8)

worin $R^7$ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

7. Mittel nach einem der Ansprüche 1 bis 6, **dadurch gekennzeichnet, dass** die besagten amphiphilen, kationischen Polymere in einer Menge von 0,05 Gew.-% bis 8,0 Gew.-%, bevorzugt von 0,1 Gew.-% bis 5,0 Gew.-%, besonders bevorzugt von 0,2 bis 2,5 Gew.%, Gew.%, jeweils bezogen auf das Gewicht des Mittels, enthalten sind.

8. Mittel nach einem der Ansprüche 1 bis 7, **dadurch gekennzeichnet, dass** $R^1$ gemäß Formel (A1) für eine Methylgruppe steht.

9. Mittel nach einem der Ansprüche 1 bis 8, **dadurch gekennzeichnet, dass** $R^2$ gemäß Formel (A2) für eine ($C_2$ bis $C_4$)-Alkylgruppe steht, insbesondere für Ethyl, n-Propyl, Isopropyl, n-Butyl oder tert-Butyl.

10. Mittel nach einem der Ansprüche 1 bis 9, **dadurch gekennzeichnet, dass** die besagten Copolymere der Komponente (b) in einer Menge von 0,05 Gel.-% bis 8,0 Gew.-%, bevorzugt von 0,1 Gew.-% bis 5,0 Gel.-%, besonders bevorzugt von 0,2 bis 2,5 Gew.%, Gel.%, jeweils bezogen auf das Gewicht des Mittels, enthalten sind.

11. Mittel nach einem der Ansprüche 1 bis 10, **dadurch gekennzeichnet, dass** es zusätzlich mindestens ein Alkanolamin enthält.

12. Verwendung eines Mittels nach einem der Ansprüche 1 bis 11 zur temporären Verformung von Haaren und/oder zur Haarpflege.

13. Verfahren zur Behandlung keratinhaltiger Fasern, insbesondere menschlicher Haare, worin unter Einsatz einer Abgabevorrichtung ein Mittel einem der Ansprüche 1 bis 11 zu einem Schaum verschäumt wird und der resultierende

Schaum auf die keratinhaltigen Fasern appliziert wird.

**14.** Verfahren zur Behandlung keratinhaltiger Fasern, insbesondere menschlicher Haare, worin unter Einsatz einer Abgabevorrichtung ein Mittel nach einem der Ansprüche 1 bis 11 als Spray auf die keratinhaltigen Fasern appliziert wird.

## Claims

**1.** A cosmetic agent for cosmetically treating keratin-containing fibers, in particular human hair, containing, in a cosmetically acceptable carrier,

(a) at least one amphiphilic, cationic polymer, comprising at least one structural unit of formula (I), at least one structural unit of formula (II), at least one structural unit of formula (III) and at least one structural unit of formula (IV),

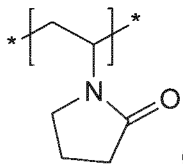 (I) 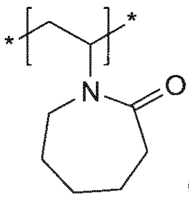 (II) 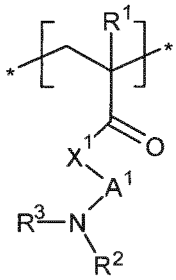 (III) 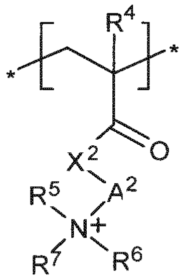 (IV)

where

$R^1$ and $R^4$ represent, independently of one another, a hydrogen atom or a methyl group,
$X^1$ and $X^2$ represent, independently of one another, an oxygen atom or an NH group,
$A^1$ and $A^2$ represent, independently of one another, an ethane-1,2-diyl group, a propane-1,3-diyl group or a butane-1,4-diyl group,
$R^2$, $R^3$, $R^5$ and $R^6$ represent, independently of one another, a ($C_1$ to $C_4$) alkyl group,
$R^7$ represents a ($C_8$ to $C_{30}$) alkyl group
and

(b) at least one copolymer comprising at least one structural unit of formula (A1) and at least one structural unit of formula (A2)

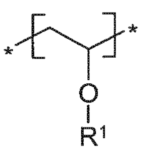 (A1) 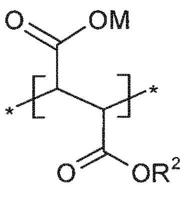 (A2)

where

$R^1$ represents a ($C_1$ to $C_{18}$) alkyl group,
$R^2$ represents a ($C_1$ to $C_6$) alkyl group,
M represents a hydrogen atom or an equivalent of a monovalent or polyvalent cation

(c) at least one propellant.

**2.** The agent according to claim 1, **characterized in that**, according to formula (III) and formula (IV), respectively, $R^1$ and $R^4$ each represent a methyl group.

3. The agent according to one of claims 1 or 2, **characterized in that**, according to formula (III) and formula (IV), respectively, $A^1$ and $A^2$ represent, independently of one another, ethane-1,2-diyl or propane-1,3-diyl.

4. The agent according to one of claims 1 to 3, **characterized in that**, according to formula (III) and formula (IV), respectively, $R^2$ and $R^3$ and $R^5$ and $R^6$ represent, independently of one another, methyl or ethyl, particularly preferably methyl.

5. The agent according to one of claims 1 to 4, **characterized in that**, according to formula (IV), $R^7$ represents a ($C_{10}$ to $C_{24}$) alkyl group, in particular decyl (caprinyl), dodecyl (lauryl), tetradecyl (myristyl), hexadecyl (cetyl), octadecyl (stearyl), eicosyl (arachyl) or docosyl (behenyl).

6. The agent according to one of claims 1 to 5, **characterized in that** the amphiphilic, cationic polymer contained therein comprises at least one structural unit of formula (II), at least one structural unit of formula (III-8) and at least one structural unit of formula (IV-8)

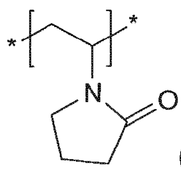 (I) 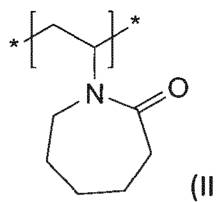 (II) 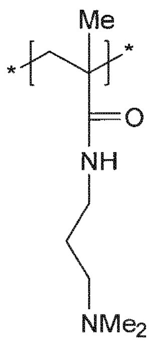 (III-8) 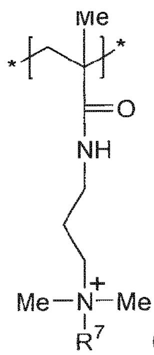 (IV-8)

where $R^7$ represents octyl (capryl), decyl (caprinyl), dodecyl (lauryl), tetradecyl (myristyl), hexadecyl (cetyl), octadecyl (stearyl), eicosyl (arachyl) or docosyl (behenyl).

7. The agent according to one of claims 1 to 6, **characterized in that** said amphiphilic, cationic polymers are contained in a quantity of from 0.05 wt.% to 8.0 wt.%, preferably from 0.1 wt.% to 5.0 wt.%, particularly preferably from 0.2 wt.% to 2.5 wt.%, in each case based on the weight of the agent.

8. The agent according to one of claims 1 to 7, **characterized in that** $R^1$ according to formula (A1) represents a methyl group.

9. The agent according to one of claims 1 to 8, **characterized in that** $R^2$ according to formula (A2) represents a ($C_2$ to $C_4$) alkyl group, in particular ethyl, n-propyl, isopropyl, n-butyl or tert-butyl.

10. The agent according to one of claims 1 to 9, **characterized in that** said copolymers of component (b) are contained in a quantity of from 0.05 wt.% to 8.0 wt.%, preferably from 0.1 wt.% to 5.0 wt.%, particularly preferably from 0.2 wt.% to 2.5 wt.%, in each case based on the weight of the agent.

11. The agent according to one of claims 1 to 10, **characterized in that** it additionally contains at least one alkanolamine.

12. The use of an agent according to one of claims 1 to 11 for temporarily shaping hair and/or for hair care.

13. A method for treating keratin-containing fibers, in particular human hair, wherein, by using a dispensing device, an agent according to one of claims 1 to 11 is foamed to form a mousse and the resulting mousse is applied to the keratin-containing fibers.

14. The method for treating keratin-containing fibers, in particular human hair, wherein, by using a dispensing device, an agent according to one of claims 1 to 11 is applied to the keratin-containing fibers as a spray.

## Revendications

1. Agent cosmétique pour le traitement cosmétique de fibres kératiniques, en particulier du cheveu humain, contenant dans un excipient cosmétiquement acceptable

(a) au moins un polymère cationique amphiphile, comprenant au moins une unité structurelle de formule (I), au moins une unité structurelle de formule (II), au moins une unité structurelle de formule (III) et au moins une unité structurelle de formule (IV),

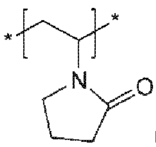 (I) 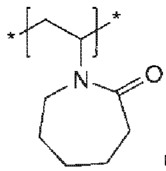 (II) 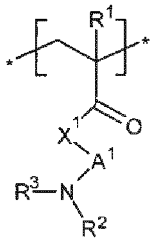 (III) 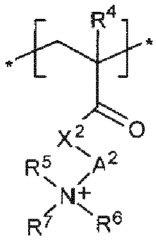 (IV)

où

$R^1$ et $R^4$ représentent indépendamment l'un de l'autre un atome d'hydrogène ou un groupe méthyle,
$X^1$ et $X^2$ représentent indépendamment l'un de l'autre un atome d'oxygène ou un groupe NH,
$A^1$ et $A^2$ représentent indépendamment l'un de l'autre un groupe éthane-1,2-diyl, propane-1,3-diyl ou butane-1,4-diyl,
$R^2$, $R^3$, $R^5$ et $R^6$ représentent indépendamment les uns des autres un groupe alkyle (en $C_1$ à $C_4$), $R^7$ représente un groupe alkyle en ($C_8$ à $C_{30}$)
et

(b) au moins un copolymère comprenant au moins une unité structurelle de formule (A1) et au moins une unité structurelle de formule (A2)

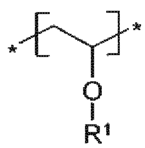 (A1) 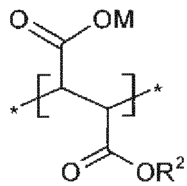 (A2)

où

$R^1$ représente un groupe alkyle (en $C_1$ à $C_{18}$),
$R^2$ représente un groupe alkyle (en $C_1$ à $C_6$),
M représente un atome d'hydrogène ou un équivalent d'un cation monovalent ou polyvalent

(c) au moins un agent propulseur.

2. Agent selon la revendication 1, **caractérisé en ce que** selon la formule (III) ou la formule (IV), $R^1$ et $R^4$ représentent chacun un groupe méthyle.

3. Agent selon une des revendications 1 ou 2, **caractérisé en ce que** selon la formule (III) ou la formule (IV), $A^1$ et $A^2$ représentent indépendamment l'un de l'autre l'éthane-1,2-diyl ou le propane-1,3-diyl.

4. Agent selon une des revendications 1 à 3, **caractérisé en ce que** selon la formule (III) ou la formule (IV), $R^2$, $R^3$, $R^5$ et $R^6$ représentent indépendamment les uns des autres le méthyle ou l'éthyle, (de manière particulièrement préférée le méthyle).

**5.** Agent selon une des revendications 1 à 4, caractérisé en que selon la formule (IV), $R^7$ représente un groupe alkyle en ($C_{10}$ à $C_{24}$), en particulier le décyle (caprinyle), dodécyle (lauryle), tétradecyle (myristyle), hexadécyle (cétyle), octadécyle (stéaryle), éicosyle (arachyle) ou docosyle (béhényle).

**6.** Agent selon une des revendications 1 à 5, **caractérisé en ce que** le polymère cationique amphiphile qui y est présent comprend au moins une unité structurelle de formule (II), au moins une unité structurelle de formule (III-8) et au moins une unité structurelle de formule (IV-8)

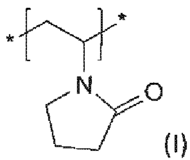 (I) 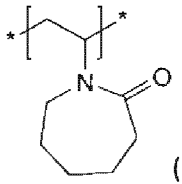 (II) 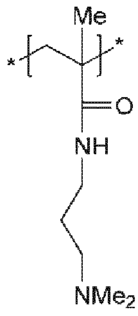 (III-8) 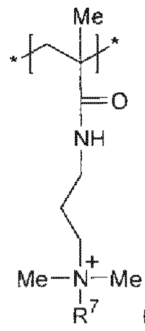 (IV-8)

où $R^7$ représente l'octyle (capryle), décyle (caprinyle), dodécyle (lauryle), tétradecyle (myristyle), hexadécyle (cétyle), octadécyle (stéaryle), éicosyle (arachyle) ou docosyle (béhényle).

**7.** Agent selon une des revendications 1 à 6, **caractérisé en ce que** lesdits polymères cationiques amphiphiles sont présents dans une proportion de 0,05 % en poids à 8,0 % en poids, de préférence de 0,1 % en poids à 5,0 % en poids, de manière particulièrement préférée de 0,2 à 2,5 % en poids, respectivement par rapport au poids de l'agent.

**8.** Agent selon une des revendications 1 à 7 **caractérisé en ce que** $R^1$ représente un groupe méthyle selon la formule (A1).

**9.** Agent selon une des revendications 1 à 8, **caractérisé en ce que** $R^2$ représente selon la formule (A2) un groupe alkyle (en $C_2$ à $C_4$), en particulier l'éthyle, le n-propyle, l'isopropyle, le n-butyle ou le tert-butyle.

**10.** Agent selon une des revendications 1 à 9, **caractérisé en ce que** lesdits copolymères du composant (b) sont présents dans une quantité de 0,05 % en poids à 8,0 % en poids, de préférence de 0,1 % en poids à 5,0 % en poids, de manière particulièrement préférée de 0,2 à 2,5 % en poids, respectivement par rapport au poids de l'agent.

**11.** Agent selon une des revendications 1 à 10, **caractérisé en ce qu'**il contient en plus au moins un alcanolamine.

**12.** Utilisation d'un agent selon une des revendications 1 à 11 pour la mise en forme temporaire des cheveux et/ou le soin des cheveux.

**13.** Procédé de traitement de fibres kératiniques, en particulier de cheveux humains dans lequel, à l'aide d'un dispositif de distribution, un agent selon une des revendications 1 à 11 est transformé en mousse et la mousse en résultant est appliquée sur les fibres kératiniques.

**14.** Procédé de traitement de fibres kératiniques, en particulier de cheveux humains, dans lequel à l'aide d'un dispositif de distribution, un agent selon une des revendications 1 à 11 est appliqué sous forme de spray sur les fibres kératiniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102008038105 A1
- WO 9922701 A1
- DE OS19738866 A
- DE OS19756454 A
- EP 998908 A2